# FASCICULE DE BREVET EUROPEEN

(11) **EP 4 062 173 B1**
(45) Date de publication et mention de la délivrance du brevet: **16.07.2025**
(21) Numéro de dépôt: 20821349.6
(22) Date de dépôt: 19.11.2020
(51) Int. Cl.: G01N 33/49, B01L 3/00

(54) **SYSTÈME DE CAPTURE ET DE DÉTECTION D'ESPÈCES PRÉSENTES DANS UN FLUIDE BIOLOGIQUE**
SYSTEM ZUM ERFASSEN UND DETEKTIEREN VON IN EINEM BIOLOGISCHEN FLUID VORHANDENEN SPEZIES
SYSTEM FOR CAPTURING AND DETECTING SPECIES PRESENT IN A BIOLOGICAL FLUID

(30) Priorité: 19.11.2019 FR 1912912
(43) Date de publication de la demande: 28.09.2022
(73) Titulaire: SMARTCATCH, 31106 Toulouse Cedex 1 (FR); Centre National de la Recherche Scientifique, 75016 Paris (FR); Institut National des Sciences Appliquées de Toulouse, 31077 Toulouse Cedex 4 (FR); Institut National Polytechnique de Toulouse, 31400 Toulouse (FR); Université Toulouse III - Paul Sabatier, 31400 Toulouse (FR)
(72) Inventeur: JIMENEZ-ZENTENO, Alejandro Kayum, 31000 TOULOUSE (FR); BOURRIER, David, 31520 RAMONVILLE ST AGNE (FR); BOU, Elise, 31400 TOULOUSE (FR); CERF, Aline, 31650 SAINT ORENS DE GAMEVILLE (FR); AUBERT, Hervé, 31000 TOULOUSE (FR); VIEU, Christophe, 31320 AUZEVILLE TOLOSANE (FR); MALAVAUD, Bernard, 31500 TOULOUSE (FR)
(74) Mandataire: Regimbeau
(86) Numéro de dépôt international: PCT/FR2020/052138
(87) Numéro de publication internationale: WO 2021/099749

(56) Documents cités:
- DE-A1- 102014 209 193
- US-A- 4 834 888
- US-A1- 2011 177 551
- ALEJANDRO KAYUM JIMENEZ ZENTENO: "Engineered micro-devices for the isolation of circulating tumor cells in clinical routine", 21 September 2018 (2018-09-21), XP055729508, Retrieved from the Internet <URL:https://hal.laas.fr/tel-02137588/document> [retrieved on 20200910]
- LIU YAOPING ET AL: "A high-throughput liquid biopsy for rapid rare cell separation from large-volume samples", LAB ON A CHIP, vol. 19, no. 1, 5 December 2018 (2018-12-05), pages 68 - 78, XP055781250, ISSN: 1473-0197, DOI: 10.1039/C8LC01048J

## Description

### DOMAINE TECHNIQUE GENERAL

L'invention concerne le domaine de la capture d'espèces spécifiques ou de particules présents dans un fluide biologique ou non (sang et ses dérivés tels que le plasma et le sérum, urine, eau, air, et tout fluide nécessitant analyse). L'invention concerne notamment la capture de cellules d'intérêts présentes dans un fluide biologique tel que le sang afin d'analyser ces cellules pour effectuer un suivi médical. L'invention trouve notamment application dans la détection de cellules tumorales circulantes pour le suivi médical d'un patient atteint d'une pathologie cancéreuse et/ou l'extraction quasi-complète des cellules tumorales contenues dans le sang, pour le diagnostic d'une pathologie cancéreuse, et pour la thérapeutique oncologique, l'invention permettant de filtrer/extraire des cellules tumorales. L'invention s'applique aussi au pronostic et au suivi de l'efficacité thérapeutique au moyen des cellules pouvant être détectées.

### ETAT DE LA TECHNIQUE

Un fluide peut être porteur de plusieurs types d'espèces ou billes dont l'énumération et l'analyse présentent un intérêt. En particulier, les fluides biologiques animaux ou humains sont porteurs de plusieurs types de cellules dont la présence peut permettre de suivre ou détecter différentes pathologies.

En particulier, les cellules tumorales circulantes (CTCs ci-après) dans le sang circulant sont étudiées pour permettre de diagnostiquer des cancers et ainsi permettre une meilleure prise en charge des patients.

En effet les tumeurs cancéreuses libèrent dans la circulation sanguine des CTCs et il est démontré que ce phénomène apparaît à un stade précoce de la maladie. L'analyse biologique et moléculaire d'une ou plusieurs cellule(s) permet de porter un diagnostic précis et d'apporter des informations notamment sur l'agressivité du cancer et l'efficacité d'un traitement. Les CTCs représentent donc un biomarqueur d'intérêt à tous les temps de la prise en charge de la maladie cancéreuse, diagnostic, pronostic et surveillance.

Or les CTCs sont présentes en concentration extrêmement faible dans le sang des patients atteints de cancer (environ 1/10⁹ cellules sanguines normales). L'isolation et/ou l'extraction quasi-complète de ces cellules rares est donc extrêmement difficile.

On connaît différents procédés *in vitro* pour procéder à cette isolation, basés sur une immunodétection. Ils se fondent sur la présence à la surface des CTCs d'EpCAM (en anglais, « *epithelial cell adhesion molecule »),* un antigène membranaire spécifique des cellules d'origine épithéliale. Un échantillon de 7,5 ml de sang est centrifugé, puis mis en présence de nanoparticules ferromagnétiques munies à leur surface d'anticorps anti-EpCAM. Les CTCs sont alors séparées des autres cellules par application d'un champ magnétique.

L'inconvénient de ce système est double :
- il utilise un échantillon de sang très limité (7,5 ml ce qui correspond à 0.15 % du volume sanguin total), dans lequel le nombre de CTCs, compte tenu de leur faible concentration, est très faible ;
- il ne permet pas de détecter les CTCs ayant perdu la protéine EpCAM lors de la transition épithélio-mésenchymateuse (EMT) ce qui représente environ 2/3 de la population totale de ces cellules ; d'autre part, il ne permet la détection que de cellules différenciées dont la durée de vie est limitée et qui ne sont pas les plus dangereuses.

On connaît d'autres approches pour isoler les CTCs *in vitro* à partir d'un échantillon sanguin, basées sur la taille des CTCs. En particulier, le système ISET (« Isolation by Size of Epithelial Tumor Cells ») utilise la filtration d'un échantillon de sang traité (lyse des globules rouges préalable) sur une membrane en polycarbonate micro perforée ; dans ce système, les CTCs sont préalablement rigidifiées par application de paraformaldéhyde de façon à résister à la forte pression qui est appliquée.

De façon générale, la sensibilité de la détection *in vitro* est réduite du fait du faible volume des échantillons. En effet, étant donné la rareté des CTCs dans le sang, leur présence dans un échantillon de quelques millilitres peut se chiffrer à quelques unités à un stade déjà avancé du cancer. Leur détection à des stades beaucoup plus précoces est de fait quasi-impossible.

Plus récemment des systèmes conçus pour être utilisés in vivo ou par aphérèse ont été développés afin d'effectuer la capture des CTCs dans le milieu physiologique, ce qui permet de préserver au mieux leur viabilité et de potentiellement accéder à des volumes sanguins plus importants que ceux analysés par les systèmes in vitro.
Bien que les systèmes soient plus avantageux que les systèmes in vitro, aucun des systèmes ne permet la capture et la numération simultanée des CTCs notamment pour une utilisation en temps réel.

En effet les systèmes connus mettent en œuvre des techniques d'immunomarquage des cellules capturées pour identifier leur nature tumorale. Ces techniques requièrent de la manipulation post-capture, sont coûteuses et ne permettent pas une détection directe en temps réel au chevet du patient. Les plateformes ainsi utilisées doivent en outre être transparentes pour permettre l'utilisation d'un microscope à fluorescence et comptabiliser les cellules capturées. De plus ces techniques ne permettent pas de garantir l'intégrité des cellules marquées qui sont altérées avant les analyses biologiques ou leur mise en culture.

Plus généralement, un dispositif de filtration d'un fluide biologique est connu du US 2011/177551 A1.

### PRESENTATION DE L'INVENTION

L'invention propose un système qui pallie les inconvénients des techniques antérieures.

A cet effet, l'invention propose, selon un premier aspect, un système de détection selon la revendication 1.

L'invention selon le premier aspect est avantageusement complétée par les caractéristiques suivantes, prises seules ou en une quelconque de leur combinaison techniquement possible :
- le système comprend une pluralité d'électrodes disposées autour dudit au moins un pore, lesdites électrodes formant un ou plusieurs circuits électriques polarisés par un signal électrique alternatif permettant la mesure des variations d'impédances complexes entre ces électrodes, dès lors qu'une ou des cellules se logent dans ou à proximité d'un pore, la mesure de la variation du module de l'impédance complexe et de sa phase permettant de discriminer le type de cellules ;
- le signal électrique alternatif appliqué aux électrodes présente une fréquence telle qu'un champ électrique crée par les électrodes permet de capturer ou libérer des cellules dans les pores grâce à la force diélectrophorétique générée entre les électrodes ;
- la fréquence de polarisation induit une force diélectrophorétique positive pendant la capture de manière à centrer les cellules entre les électrodes et à retenir les cellules dans les pores ;
- la fréquence de polarisation induit une force diélectrophorétique négative permettant de détacher toutes les cellules capturées, la fréquence étant typiquement de 1MHz ;
- la fréquence induit une force diélectrophorétique permettant de détacher sélectivement un type de cellule, la fréquence étant comprise entre 50 kHz et 150kHz, de préférence 100kHz pour détacher les cellules tumorales ;
- la fréquence de polarisation est augmentée par palier entre 10kHz et 200 kHz de manière à détacher des cellules à des instants différents en fonction de leurs propriétés diélectriques ;
- le système comprend en outre une inductance connectée aux électrodes de manière à former un circuit résonateur électromagnétique, les électrodes et l'inductance formant un circuit de détection, de préférence interrogeable à distance, de la présence de cellules piégées ;
- la membrane filtrante est en matériau choisi du groupe comprenant du verre ou du métal (Nickel, Or) ou polymère, matériau ferromagnétique, matériau magnétique (NiFe), ou la combinaison de verre et de Silicium ou de Nickel et de Silicium, Nitrure de Silicium, Oxyde de silicium, Silicium ou plus généralement d'un matériau biocompatible et non toxique ;
- les pores présentent une dimension transversale comprise entre 0,1 µm et 100 µm, de préférence entre 8 µm et 12 µm ou entre 8 µm et 15 µm ; et/ou les pores sont espacés d'un intervalle compris entre 100 nm et 100 µm ; le nombre de pores est compris entre 100 et 100 000 000 ;
- les pores sont sensiblement circulaires ou sensiblement ovales ou sensiblement polygonaux ou prennent la forme d'une fente ;
- les pores de la membrane sont arrangés par groupe de plusieurs pores, chaque groupe présentant un motif, les groupes pouvant être reliés entre eux via une rangée de pores ;
- le motif formé par un groupe présente une forme : hexagonale, circulaire ;
- la membrane comprend plusieurs groupes de pores arrangés selon une structure en carré ou en forme d'étoile ;
- les pores de la membrane sont arrangés de manière aléatoire ;
- les moyens de filtration comprennent un support plan comprenant une zone évidée où se situe la membrane filtrante et dans lequel ladite ouverture est formée, ladite ouverture étant disposée en périphérie de la membrane filtrante ;
- le système comprend un compartiment dans lequel sont logés les moyens de filtration, le compartiment comprenant un module d'entrée et un module de sortie assemblés entre eux pour permettre de faire un circuler un fluide depuis le module d'entrée vers le module de sortie en passant par les moyens de filtration ;
- le système comprend un rack d'entrée et un rack de sortie, une lame supportant les moyens de filtration, le rack d'entrée et le rack de sortie étant assemblés entre eux de telle sorte que la lame soit entre le rack d'entrée et le rack de sortie pour permettre de faire un circuler un fluide depuis le rack d'entrée vers le rack de sortie en passant par les moyens de filtration.

L'invention selon le deuxième aspect propose un ensemble de capture comprenant une pluralité de systèmes selon l'invention disposés en série, chaque système comprenant des moyens de filtration adaptés pour retenir un type d'espèce. L'invention selon le deuxième aspect est avantageusement complétée par les caractéristiques suivantes, prises seules ou en une quelconque de leur combinaison techniquement possible :
- l'ensemble comprend un module d'entrée, un module de sortie, et au moins un module intermédiaire disposé entre le module d'entrée et le module de sortie, le module intermédiaire et le module de sortie supportant des moyens de filtration, les dits modules comprenant des moyens de fixation entre eux ;
- le module d'entrée comprend une entrée du fluide et le module de sortie comprend une sortie du fluide ;
- l'ensemble comprend un rack d'entrée, un rack de sortie, et au moins un rack intermédiaire disposé entre le rack d'entrée et le rack de sortie, le rack intermédiaire et le rack de sortie supportant des moyens de filtration, lesdits racks comprenant des moyens de fixation entre eux pour former un ensemble unitaire.

L'invention concerne également un procédé de capture de cellules circulantes dans un fluide selon la revendication 10.

Le procédé selon l'invention est avantageusement complété par les caractéristiques suivantes, prises seules ou en une quelconque de leur combinaison techniquement possible :
- la fréquence de polarisation induit une force diélectrophorétique positive pendant la capture de manière à centrer les cellules entre les électrodes et à retenir les cellules dans les pores ;
- la fréquence de polarisation induit une force diélectrophorétique négative permettant de détacher toutes les cellules capturées, la fréquence étant typiquement de 1MHz ;
- la fréquence induit une force diélectrophorétique permettant de détacher sélectivement un type de cellule, la fréquence étant comprise entre 50 kHz et 150kHz, de préférence 100kHz pour détacher les cellules tumorales ;
- la fréquence de polarisation est augmentée par paliers entre 10kHz et 200 kHz de manière à détacher les cellules capturées à des instants différente en fonction de leurs propriétés diélectriques.

La capture de différentes espèces se base sur les propriétés physiques des espèces circulant dans un fluide et notamment sur leur taille et leur déformabilité. Dans le cas de cellules le fluide est biologique (sang, urine, lymphe et de manière plus générale tout fluide circulant dans un être humain ou un animal et portant des cellules ayant un intérêt à être analysées). Ce fluide biologique peut ou ne pas être dilué dans une solution tampon. Ce fluide peut également être un milieu de culture.

S'agissant de cellules potentiellement présentes dans le sang, les plaquettes ont une dimension de 2 à 4 µm et les globules rouges une dimension d'environ 7µm ; les globules blancs ont des dimensions variables de 7 à 15 µm mais ils sont très déformables. Les CTCs ont des dimensions variables, comprises entre 4 et 25 µm mais elles sont peu déformables.

De ce fait, dès lors que les cellules sont peu déformables, elles peuvent être capturées par les moyens de filtration tout en laissant passer les autres composants non tumoraux du fluide biologique dans des conditions normales de l'écoulement sanguin tel qu'il existe in vivo.

La présence des ouvertures permet d'assurer une continuité de circulation du fluide biologique dans les conditions in vivo de pression et de vitesse et ce quel que soit le remplissage de la membrane filtrante par les éléments capturés.

De manière préférée, en couplant la membrane filtrante et ses orifices à des électrodes, l'invention combine la capture physique à la détection des cellules piégées au voisinage des pores. Ainsi, la numération des cellules capturées peut être effectuée en temps réel.

En particulier, lorsqu'il s'agit de CTCs ces dernières ayant des propriétés diélectriques spécifiques, elles influent sur l'impédance du circuit électrique formé par les électrodes. Elles influent en temps réel sur un signal électrique et permettent donc une détection en temps réel des cellules capturées par le moyen de filtration ou du taux d'obstruction des moyens de filtration. La détection du taux d'obstruction permet de déterminer si les moyens de filtration peuvent permettre la capture d'espèces sans être saturés.
Partant du constat que les CTCs présentent des propriétés diélectriques différentes des autres cellules qui pourraient être piégées, il est possible de les discriminer parmi les cellules capturées.

La détection électrique en temps réel des CTCs basée sur leurs propriétés diélectriques permet de s'affranchir des étapes de marquage immunologique des cellules tumorales et de la mise en place d'un système compatible avec la microscopie optique nécessaire pour la caractérisation des cellules marquées. Ainsi, l'invention permet de caractériser directement la présence de CTCs sur la membrane filtrante en évitant la manipulation ultérieure du dispositif. L'information médicale est donc délivrée instantanément, à moindre coût et de façon non invasive. Le suivi en temps réel de la capture présente un avantage important pour les utilisations in vivo et ex vivo en permettant à l'utilisateur d'ajuster le temps d'exposition du dispositif au fluide sanguin et à la charge tumorale du patient analysé, afin de personnaliser le temps d'exposition du dispositif en fonction de la richesse ou de la rareté de l'information du dispositif et donc la précision de l'information médicale fournie. En outre, l'invention permet également de retirer, après capture, les cellules tumorales circulantes contenues dans le sang offrant une modalité thérapeutique.

Grâce à l'invention, on offre au clinicien une information initiale immédiate sur le nombre d'espèces par unité de temps ou de volume exposé qui sera ensuite complétée par l'analyse de la population ainsi capturée. Soulignons que de nombreuses méthodes analytiques demandent une prise d'essai minimale et que l'invention permet aussi de s'assurer que les conditions de réalisations des analyses sont bien remplies avant d'engager des dépenses souvent coûteuses. En outre, on s'affranchit de toute étape de recueil, d'acheminement et de préparation de l'échantillon. La qualité de l'information est préservée, les cellules sont isolées en conditions natives et physiologiques, et cette information est restituée sur-le-champ au spécialiste, au sein-même du lieu de consultation, pour une prise de décision immédiate. L'invention permet en outre de générer des données utiles à la prise en charge du patient. Les conditions de fluide sanguin variant d'un patient à l'autre et à l'échelle du patient lui-même tout au long de la journée, cette invention peut être couplée à une mesure de vitesse de fluide locale qui servira de calibration ou de référence pour constituer une base de données comparables.

Avantageusement, grâce au couplage du circuit électrique formé par les électrodes à un circuit résonateur, les variations d'impédance liées à la détection des CTCs peuvent être mesurées à distance et sans contact dans un mode sans fil (en anglais, « *remote wireless* »).

Également, une fonctionnalisation de la surface du système avec des anticorps est envisageable et permet d'associer capture physique et capture par affinité.

La libération des cellules capturées du dispositif filtrant à des fins de récupération pour analyse ou pour remise en culture peut être réalisée par stimulation électrique grâce aux mêmes électrodes ayant permis la détection.

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre un système de capture d'espèces présentes dans un fluide ;
- la figure 2 illustre une membrane filtrante selon un mode de réalisation de l'invention ;
- la figure 3 un schéma plus détaillé de la membrane de la figure 2 ;
- les figures 4a, 4b, 5 et 6 illustrent un schéma des membranes filtrantes selon différents modes de réalisation ;
- la figure 7 illustre deux modes de réalisation pour l'agencement des électrodes autour des orifices des membranes filtrantes selon l'invention ;
- la figure 8 illustre un compartiment du système de l'invention ;
- les figures 9a, 9b, 9c, 9d, 9e illustrent différentes vues d'un module d'entrée d'un compartiment du système de l'invention ;
- les figures 10a, 10b, 10c, 10d, 10e, 10f illustrent différentes vues d'un module de sortie d'un compartiment du système de l'invention ;
- les figures 11a, 11b illustrent la fixation d'un système de capture dans un module du compartiment du système selon l'invention ;
- les figures 12a, 12b et 12c illustrent le compartiment de la figure 8 avec en plus un module intermédiaire ;
- les figures 13a, 13b, 13c, 13d, 13e, 13f illustrent différentes vues d'un module intermédiaire d'un compartiment du système de l'invention ;
- les figures 14 à 17 illustrent un rack et des associations de racks selon un mode de réalisation de l'invention ;
- les figures 18 à 20 illustrent des racks assemblés entre eux selon un mode de réalisation de l'invention ;
- la figure 21 illustre schématiquement des étapes d'un procédé de capture et de détection selon l'invention.

Sur l'ensemble des figures les éléments similaires portent des références identiques.

### DESCRIPTION DETAILLEE

On décrit ci-après un mode de réalisation de l'invention dans le cadre de la capture et de la détection de cellules présentes dans un fluide biologique mais l'invention s'applique à la capture et à la détection de tous types d'espèces ou de billes présentes dans un fluide biologique ou non (sang et ses dérivés tels que le plasma et le sérum, urine, eau, air, polluant, etc.).

On précise que l'invention s'applique à la capture d'espèces et qu'on entend par espèce : une cellule tumorale ; un agrégat de cellules tumorales ; un caillot de sang (le fluide étant alors du sang) ; un exosome.

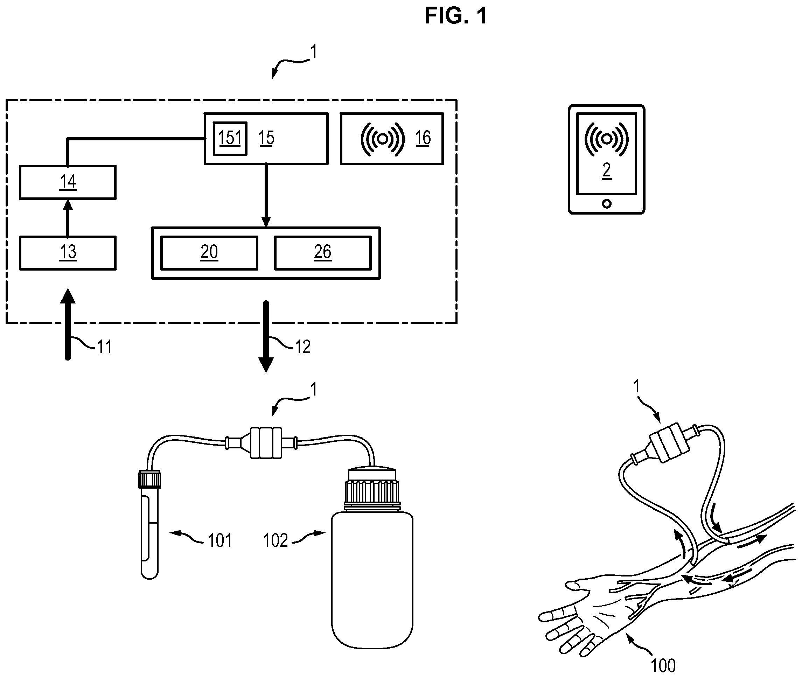

En relation avec la **figure 1**, un système 1 de capture de cellules circulantes présentes dans un fluide comprend une entrée 11 de fluide et une sortie 12 de fluide. Il doit être compris qu'un fluide est en circulation et passe au travers du système ici décrit.

Un tel système 1 peut être relié à la circulation sanguine d'un humain via son bras 100 mais il peut bien entendu être également relié à une autre partie du corps humain ou d'un animal. De préférence, le système sera connecté à une veine périphérique (par exemple dans le pli du coude) ou à une voie veineuse centrale d'un humain ou d'un animal. Alternativement, un tel système 1 peut être relié d'une part à une éprouvette 101 contenant un fluide et d'autre part à un système 102 de récupération du fluide après passage dans le système 1 de capture.

Dans le cas d'une utilisation ex vivo ou in vitro une pompe péristaltique ou contrôlée en pression 13 est reliée à l'entrée du fluide, un capteur 14 de débit permet de régler la pompe 13. La pompe 13 permet d'amener le fluide aux moyens de filtration 20 qui vont être décrits ci-après en détail. Ainsi, le fluide entrant passe au travers des moyens de filtration 20 et sont réinjectés dans le corps du patient ou de l'animal ou bien dans un tube de récupération du fluide.
L'entrée de fluide et la sortie de fluide sont reliées au fluide circulant par des cathéters ou par tout autre moyen connu de l'homme du métier et adaptés à l'endroit où le système doit être utilisé.

Alternativement, le système peut être utilisé in vivo et ne nécessite pas de pompe. Il est dans ce cas placé directement au sein du fluide en circulation.
Les moyens de filtration 20 sont avantageusement couplés à des moyens de détection 26 des cellules capturées.

Une unité de mesure 15 en liaison avec les moyens de détection 26 permet de mesurer des informations relatives aux cellules détectées.

Le système 1 comprend également une interface de communication 16 filaire ou sans fil en communication avec un terminal 2 filaire ou sans fil. Un tel terminal 2 comprend des interfaces permettant à un utilisateur d'accéder à diverses informations relatives à la détection.

### Moyens de filtration

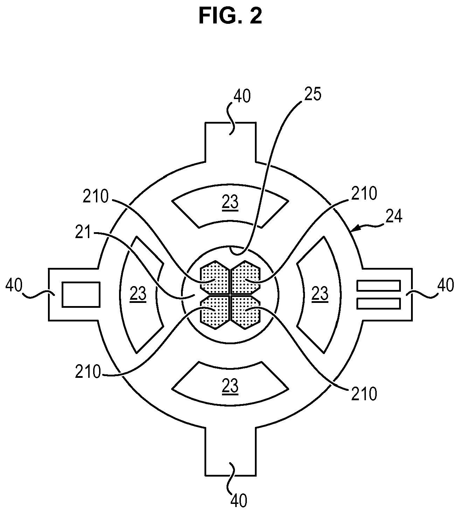

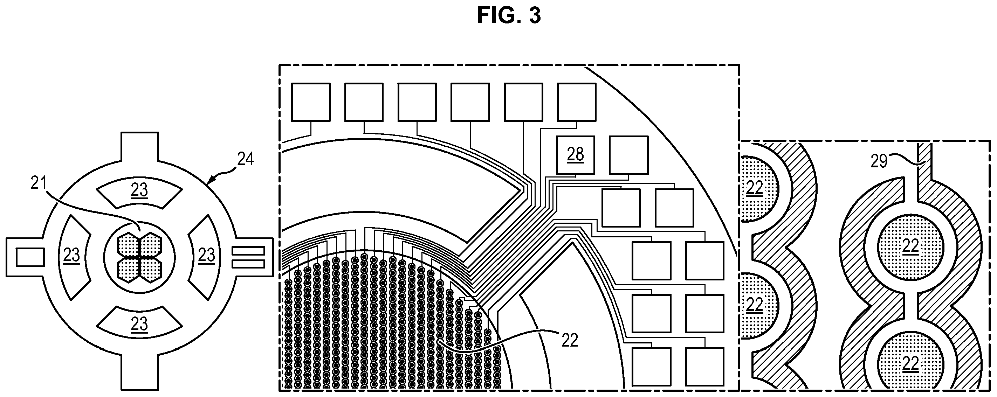

Les **figures 2** et **3** illustre une réalisation possible des moyens de filtration 20. Ces moyens de filtration monolithiques comprennent un support plan 24 par exemple de forme circulaire comprenant une zone 25 dans laquelle se situer une membrane 21 filtrante.
La membrane est par exemple de forme circulaire et se situe dans une zone 25 de la même forme.

Dans la membrane sont formés au moins un pore 22 permettant de capturer/retenir des cellules présentes dans le fluide biologique dans lequel sont placés les moyens de filtration 20. Le fluide circule au travers du système.

La membrane est constituée d'un matériau biocompatible et non toxique choisi du groupe comprenant du verre ou du métal (Nickel, Or), ou polymère, matériau ferromagnétique, matériau magnétique (NiFe), multi-matériaux (Verre/Silicium), Nitrure de Silicium, Oxyde de Silicium, Silicium.

Bien entendu pour des applications où le fluide n'est pas biologique le fait que le matériau soit biocompatible est sans importance.

Les pores présentent avantageusement une dimension transversale comprise entre 0,1 µm et 100 µm, de préférence entre 8 µm et 12 µm ou entre 8 µm et 15 µm et sont typiquement en nombre compris entre 1000 et 6000, de préférence entre 100 et 100 000 000. La taille et le nombre de pores dépend du type de cellule circulante à capturer et de la manière d'utiliser les moyens de filtration (*in vivo, ex vivo, in vitro*)*.*

De manière complémentaire, les pores sont espacés d'un intervalle compris entre 0,1 µm et 100 µm, de préférence entre 8 µm et 12 µm ou entre 8 µm et 15 µm

Les pores sont de diverses formes. Ils peuvent être sensiblement circulaires ou sensiblement ovales ou sensiblement polygonaux ou peuvent prendre la forme d'une fente.

De manière avantageuse, les pores de la membrane sont arrangés par groupe de plusieurs pores, chaque groupe présentant un motif, les groupes pouvant être reliés entre eux par l'intermédiaire d'une rangée de pores.

Par exemple, sur la figure 2, les pores sont arrangés selon quatre groupes 210, chaque groupe présentant une forme polygonale.

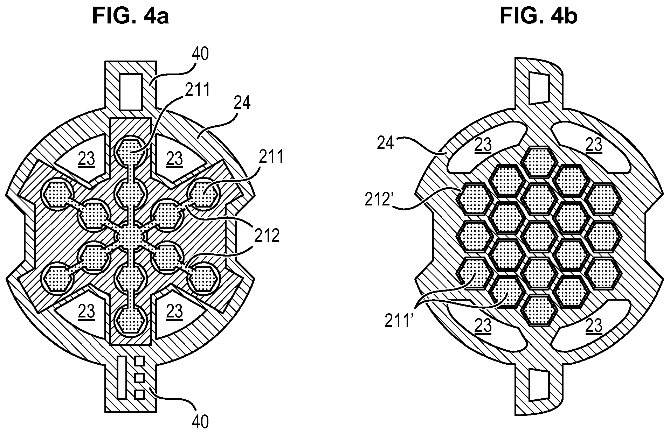

Les groupes peuvent être disposés sur la membrane selon plusieurs motifs : en carré (figure 2) ou en étoile (**figure 4a**)**.** Pour la structure en forme d'étoile les pores sont arrangés en groupes 211 de forme hexagonale, les groupes 211 de forme hexagonale sont reliés entre eux par des groupes 212 de pores en forme de fente. Ainsi, la structure en étoile est obtenue par la combinaison de deux regroupements de pores. La structure en étoile s'étend depuis un groupe 211 central disposé au centre la membrane et six branches s'étendent depuis ce groupe central. Sur chaque branche, deux groupes de forme polygonale sont reliés au groupe central, des fentes 212 connectent les groupes de forme polygonale. Le nombre de branches peut évidemment varier tout comme le nombre de groupes par branche.

De manière alternative comme illustré en relation avec la **figure 4b****,** les pores arrangés en groupe 211' ne sont pas reliés entre eux mais sont dans emplacements 212' indépendants les uns des autres. Ceci permet de maximiser le nombre de pores sur le support 24.

Afin de permettre une continuité dans la circulation du fluide, des ouvertures 23 sont formées dans le support en périphérie des pores. Afin de maintenir un écoulement sanguin non perturbé et ce quel que soit le degré d'occultation des zones filtrantes par les éléments capturés.

En faisant à nouveau référence aux figures 2 et 3, les ouvertures sont en forme de secteur angulaire autour de la membrane. Une telle forme n'est pas limitative et d'autres formes peuvent être envisagées. Également, des éléments perturbateurs du fluide ou de concentration du fluide peuvent être disposés.

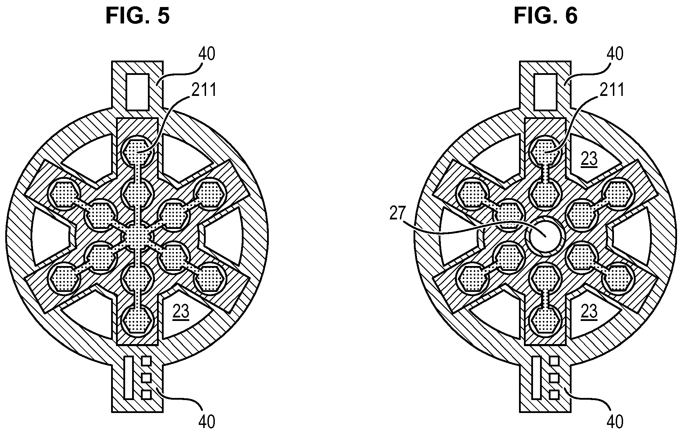

Le nombre et la forme des ouvertures sont optimisés pour altérer le moins possible la tenue mécanique des moyens de filtration 20. Sur les exemples illustrés sur les figures 2 et 3, quatre ouvertures 23 sont présentes tandis que sur l'exemple de la **figure 5****,** six ouvertures 23 sont présentes.

De manière alternative et en relation avec la **figure 6****,** la membrane peut comprendre outre, les ouvertures 23 périphériques en forme de secteur angulaire, une ouverture centrale 27. Dans cet exemple, les pores sont par groupes de forme polygonale reliés deux à deux par des groupes en forme de fente. Les ensembles de deux groupes hexagonaux sont répartis en forme d'étoile autour de l'ouverture centrale 24.

Pour la configuration de la figure 4 on peut avoir les paramètres suivants :
- Diamètre pores circulaires 8 µm, 9 µm, 10 µm, 11 µm, 12 µm ;
- Distance inter-pores de 5 µm ;
- Nombre de pores : 4246, 3395, 2717, 2287, 1880 ;
- Nombre moyen de pores par groupe hexagonal : 271, 220, 169, 135, 121 ;
- Nombre moyen de pores par fente : 54, 45, 40, 27 ;
- Surface occupée par les pores : 0,213 mm², 0,216 mm², 0,217 mm² ;
- Surface d'une ouverture : 0,111 mm² ;
- Surface totale occupée par les quatre ouvertures : 0, 444 mm².

Pour la configuration de la figure 5, on peut avoir les paramètres suivants :
- Diamètre pores circulaires 8 µm, 9 µm, 10 µm, 11 µm, 12 µm ;
- Distance inter-orifices de 5 µm ;
- Nombre de pores : 3395, 4246, 2717, 2287, 1880 ;
- Nombre moyen de pores par groupe hexagonal : 220, 271, 121, 169, 135 ;
- Nombre moyen de pores par fente : 45, 54, 40, 27, 54 ;
- Surface occupée par les pores : 0,216 mm², 0,213 mm², 0,217 mm² ;
- Surface d'une ouverture : 0,111 mm²
- Surface totale occupée par les six ouvertures : 0,666 mm².

Pour la configuration de la figure 6, on peut avoir les paramètres suivants :
- Diamètre pores 8 µm, 9 µm, 10 µm, 11 µm, 12 µm ;
- Distance inter-pores de 5 µm ;
- Nombre de pores : 1227, 486, 386, 324, 271 ;
- Nombre moyen de pores par groupe hexagonal : 220, 169, 135, 121 ;
- Nombre moyen de pores par fente : 45, 40, 27 ;
- Surface occupée par les pores : 0,213 mm², 0,216 mm², 0,217 mm² ;
- Surface occupée par l'ouverture centrale : 0,057 mm² ;
- Surface d'une ouverture périphérique : 0,111 mm² ;
- Surface totale occupée par les six ouvertures et l'ouverture centrale : 0,723 mm².

### Capture et détection et unité de mesure

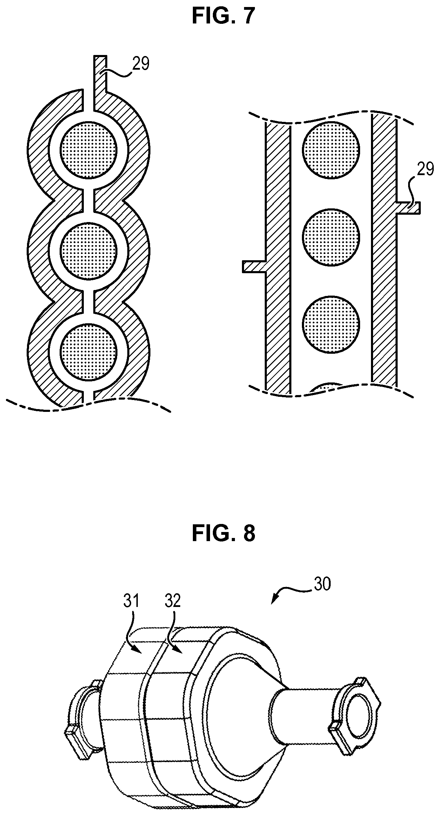

En relation avec les **figures 3** et **7** et afin de détecter les cellules capturées par les pores, par les moyens de filtration 20, le système de capture et de détection comprend avantageusement des moyens de détection 26 constitués par des électrodes disposées autour des pores 22. Les électrodes 26 sont connectées entre elles afin de former un ou plusieurs circuits électriques caractérisés par une impédance complexe qui dépend de la fréquence de l'excitation électrique. La valeur de cette impédance en fonction de la fréquence est influencée par la présence de cellules au voisinage des électrodes contournant les pores 22.

En particulier, une tension alternative est appliquée sur les électrodes à une fréquence donnée de sorte qu'une impédance complexe est mesurée entre les électrodes. La partie réelle de l'impédance mesurée est caractéristique de la résistance électrique du milieu occupant cet espace. La partie imaginaire quant à elle reflète les propriétés diélectriques de ce milieu et notamment sa permittivité. Lors de la mesure électrique nous mesurons le module de l'impédance complexe et le déphasage du courant électrique vis à vis de la tension appliquée. Ces deux mesures sondent à la fois la partie réelle et la partie imaginaire de l'impédance électrique.

Lorsqu'une cellule se positionne entre les électrodes, l'impédance électrique ainsi mesurée (par son module et sa « phase ») est modifiée. La partie réelle de l'impédance est modifié ainsi que la partie imaginaire. Nous mesurons donc ces deux changements que nous restituons par la variation du module de l'impédance et par la variation du déphasage courant/tension.

Le mesure de l'impédance complexe permet de discriminer le type cellulaire en particulier tumoral/non tumoral. C'est en effet la variation de la partie imaginaire qui contient l'information la plus précieuse afin de réaliser ce type de détection.

En particulier, s'agissant de CTCs possédant des propriétés diélectriques spécifiques, elles font varier la valeur de cette impédance de manière discernable des autres types cellulaires potentiellement piégés. Selon la variation détectée, il est possible de détecter les cellules capturées et leur type. Les électrodes sont par exemple en or, en cuivre, en platine, nickel, matériaux piézoélectriques, polymère conducteur. L'impédance est mesurée sur une gamme variant de 10 Hz à 1MHz voire 5 MHz.

Ces électrodes entourent donc les pores de la membrane filtrante de manière à sonder électriquement les propriétés diélectriques du milieu au voisinage des pores où les cellules capturées se trouvent piégées.

La partie imaginaire de l'impédance électrique, dans les gammes de fréquence du signal ci-dessus mentionnées, est dominée par la capacité formée par la membrane plasmique de la cellule. Cette bicouche lipidique riche en protéines membranaires est un excellent isolant électrique séparant deux milieux conducteurs que sont le milieu intra-cellulaire (le cytoplasme) et le milieu extra-cellulaire (le fluide à analyser contenant les cellules), formant ainsi une sorte de condensateur électrique décrit par une capacité, c'est à dire par une impédance imaginaire d'origine capacitive. En l'absence de cellule entre les électrodes cette capacité membranaire n'est pas présente dans le circuit électrique, lorsqu'une cellule se positionne entre les électrodes (dans la région où le champ électrique émanant des électrodes est présent) en revanche, cette capacité membranaire apparaît dans le circuit. Cette modification entraine une variation de la partie imaginaire de l'impédance mesurée. Bien sûr en même temps, la partie réelle de l'impédance est également modifiée. Ainsi les deux parties (réelles et imaginaires) de l'impédance qui sont modifiées par la présence de la cellule sont mesurées pendant la capture. Les cellules tumorales circulant dans le sang, présentent une morphologie de leur membrane plasmique tout à fait spécifique, se traduisant par l'existence de nombreuses protubérances qui sont absentes des cellules saines. Ces protubérances accroissent considérablement la surface de la membrane plasmique des cellules tumorales lorsqu'on la compare à la surface plasmique de cellules saines. La capacité membranaire de la cellule se comporte donc comme un condensateur plan dont la capacité est proportionnelle à la surface des conducteurs en regard. On comprend ainsi pourquoi la capacité membranaire des cellules tumorales est beaucoup plus grande que la capacité membranaire des cellules saines. Ainsi, la détection mise en oeuvre permet non seulement de détecter les cellules retenues par le dispositif de capture, mais également de pronostiquer la nature saine ou tumorale de chaque cellule capturée, grâce à l'ampleur de la modification de la partie imaginaire de l'impédance électrique.

Comme illustré sur la figure 7, les électrodes peuvent entourer de diverses manières les pores : en enveloppant les pores ou bien en formant des pistes droites autour des pores. Le choix de la forme des électrodes dépend de la densité des pores. De manière alternative, les électrodes peuvent se situer sur des parois internes des pores.

Des pistes 29 de connexion reliant les électrodes à des plots 28 de contact sont nécessaires pour effectuer des mesures directes (actives). Les plots 28 sont connectés à une unité de mesure 15 qui permet de mesurer directement la valeur des variations d'impédance induites par la présence des cellules entre les électrodes.

L'unité de mesure peut comprendre une bobine 151 connectée aux électrodes de façon à former un résonateur électromagnétique pour effectuer des mesures passives et sans fil (interrogation du dispositif et réception du signal à distance). Un terminal 2 sans fil permet alors de mesurer sans contact la variation de l'impédance des circuits de détection. La bobine 151 peut aussi être incluse dans la membrane, la valeur de son inductance pouvant alors être mesurée à distance.

Encore alternativement, une liaison filaire entre les électrodes et l'unité de mesure 15 est possible et permet directement de lire la valeur des impédances des circuits de détection.

Le terminal 2 peut aussi se connecter de manière filaire à l'unité de mesure 15.

Quelle que soit la manière de mesurer la variation d'impédance, cette mesure est directe et est en temps réel. Elle permet donc de déterminer la présence de cellules sur la surface de la membrane. Cette variation d'impédance induite par la présence de cellules au voisinage des pores de capture dépend de la nature des cellules piégées, sa mesure permet donc de discerner parmi toutes les cellules piégées lesquelles sont tumorales. Cette mesure est non invasive pour les cellules à détecter et n'affecte en aucun cas leur viabilité.

### Capture et détachement

Le système de détection est avantageusement basé sur la mise en œuvre de la force diélectrophorétique qui permet en association avec les pores de maintenir les cellules mais aussi de les détacher éventuellement sélectivement.

En effet, un objet diélectrique comme les cellules, baignant au sein d'un milieu où existe un champ électrique non uniforme (on parle alors d'un gradient du module du champ électrique) est soumis à une force capable de le mettre en mouvement en raison de sa polarisabilité.

Cette force est la base du principe de la diélectrophorèse. En présence d'un champ électrique non uniforme alternatif, la direction de cette force vis-à-vis du gradient du carré du module du champ électrique, dépend de la fréquence du champ alternatif et des propriétés diélectriques de l'objet. Pour un objet de taille et de permittivité donnés, en fonction de la fréquence du champ, la force diélectrophorétique peut être positive (l'objet diélectrique se met en mouvement vers les régions où le module du champ électrique est le plus fort) ou bien négative (l'objet diélectrique se met en mouvement vers les régions où le module du champ électrique est le plus faible).

Comme décrit ci-avant, lors de la capture cellulaire, une tension alternative est appliquée aux électrodes de manière à les détecter en temps réel. La configuration des électrodes sous forme planaire conduit donc à la génération d'un champ alternatif non uniforme au-dessus des électrodes, face au fluide à analyser.

Les régions de champ électrique fort sont proches des pores du dispositif de capture, les régions de champ électrique faible sont plus loin dans le fluide au-dessus des microélectrodes. Ces phénomènes électrocinétiques liés à la force diélectrophorétique indiquent donc que les cellules arrivant au voisinage des pores seront soumises à des forces qui peuvent soit les diriger vers les pores de capture (diélectrophorèse positive) soit les repousser (diélectrophorèse négative).

La fréquence pour laquelle la force diélectrophorétique change de signe est appelée fréquence de coupure. Un objet diélectrique en fonction de sa forme, de sa taille et de ses propriétés diélectriques (sa permittivité relative) présente une fréquence de coupure qui lui est propre.

Dans le cas de cellules en circulation dans le sang, la fréquence de coupure des cellules sanguines saines se situe autour de 150 kHz, alors que celle de cellules de lignée tumorale est significativement plus basse (50 kHz). Ceci résulte une fois de plus de la différence des propriétés diélectriques des cellules tumorales liées dans cette gamme fréquentielle à une capacité membranaire élevée. Ainsi il apparait que en polarisant les électrodes à une fréquence intermédiaire entre ces deux fréquences on peut sélectivement diriger préférentiellement les cellules d'intérêt dans une direction privilégiée de l'espace.

Par conséquent, il est possible d'utiliser le système de détection selon plusieurs configurations qui permettent de combiner en même temps la détection par mesure de l'impédance électrique et l'application d'une force qui peut conduire soit à mieux ancrer les cellules sur les microélectrodes soit à les détacher.

Pour la capture, on considère qu'un fluide circule dans un sens tandis que pour la récupération un fluide circule dans un sens opposé à celui de la capture.

La première configuration est la capture. Pendant la capture le choix d'une fréquence de polarisation induisant une force diélectrophorétique positive permet une meilleure localisation des cellules sur les électrodes facilitant ainsi leur détection électrique. Les cellules retenues par les pores se trouvent ainsi toujours localisées de la même manière conduisant à une très grande reproductibilité des mesures d'impédance électrique. Il s'agit à ce stade de ne pas exercer toutefois une force trop importante qui retiendrait toutes les cellules transitant par les pores.

La deuxième configuration est la récupération des cellules après la capture. Après la capture, la tension de polarisation est augmentée (le gradient du module du champ électrique est plus élevé), la force diélectrophorétique exercée est désormais beaucoup plus forte, la fréquence est ajustée à une valeur élevée (1MhZ) de manière à détacher toutes les cellules capturées en vue de leur récupération par diélectrophorèse négative. Pendant ce détachement électrique un écoulement fluidique (opposé à celui de la capture) est appliqué afin de collecter les cellules qui se détachent dans un réservoir. Pendant cette étape la nature du fluide circulant peut-être choisie afin de préserver la viabilité cellulaire tout en maximisant la force diélectrophorétique de détachement.

La troisième configuration est le détachement sélectif des cellules. Après capture la tension de polarisation est augmentée (le gradient du module du champ électrique est plus élevé), la fréquence est désormais ajustée de manière à détacher sélectivement un seul type cellulaire en se positionnant à une fréquence intermédiaire entre 50 kHz et 150 kHz. Pendant ce détachements électrique un écoulement fluidique (opposé à celui de la capture) est appliqué afin de collecter les cellules qui se détachent dans un réservoir spécifique. Pendant cette étape la nature du fluide circulant peut-être choisie afin de préserver la viabilité cellulaire tout en maximisant la force diélectrophorétique de détachement. Il est alors possible de détacher sélectivement et séquentiellement les différents types cellulaires. A 100 kHz seules les cellules tumorales sont détachées et collectées.

La quatrième configuration est le détachement et classement des cellules. Après capture la tension de polarisation est augmentée (le gradient du module du champ électrique est plus élevé), la fréquence est désormais augmentée par paliers entre 10 kHZ et 200 kHz de manière à détacher les cellules à des instants différents en fonction de leurs propriétés diélectriques. Pendant ces détachements électriques un écoulement fluidique (opposé à celui de la capture) est appliqué afin de collecter les cellules qui se détachent séquentiellement. Pendant cette étape la nature du fluide circulant peut-être choisie afin de préserver la viabilité cellulaire tout en maximisant la force diélectrophorétique de détachement. Les cellules détachées sont récupérées au sein d'un canal dont la section ne permet le transit qu'un d'une seule cellule à la fois. Les cellules se trouvent alors ordonnées en file avec une position qui dépend de leur fréquence de coupure diélectrophorétique et donc de leurs propriétés diélectriques. Dans cette file les cellules seront donc ordonnées en fonction de leur capacité membranaire et ainsi les cellules saines seront en queue de la file et les cellules tumorales à l'autre extrémité (en tête de file) avec toutes les gradations possibles entre ces deux extrémités.

La cinquième configuration est la récupération du lysat d'intérêt. Après la capture, la tension de polarisation est fortement augmentée (le gradient du module du champ électrique est encore plus élevé), la force diélectrophorétique exercée est très intense, la fréquence est ajustée à une valeur basse (10 kHz) une lyse cellulaire se produit relarguant ainsi le contenu des cellules capturées. Pendant cette lyse cellulaire un écoulement fluidique (opposé à celui de la capture) est appliqué afin de collecter le lysat cellulaire dans un réservoir.

### Compartiment du premier type

Selon un mode de réalisation, et comme illustré sur la **figure 8****,** le système de capture et de détection comprend avantageusement un compartiment 30 dans lequel sont logés les moyens de filtration 20. Ce compartiment comprend un module d'entrée 31 formant une partie femelle et un module de sortie 32 formant une partie mâle 32. Ainsi, le module d'entrée et le module de sortie peuvent être assemblés en les vissant ensemble ou bien en les emboitant.

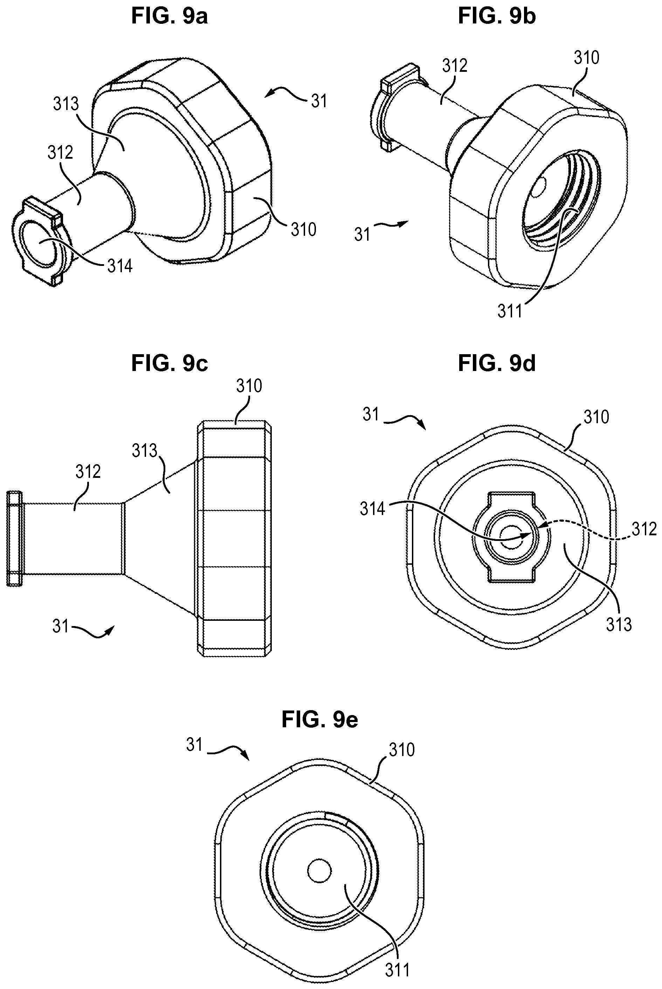

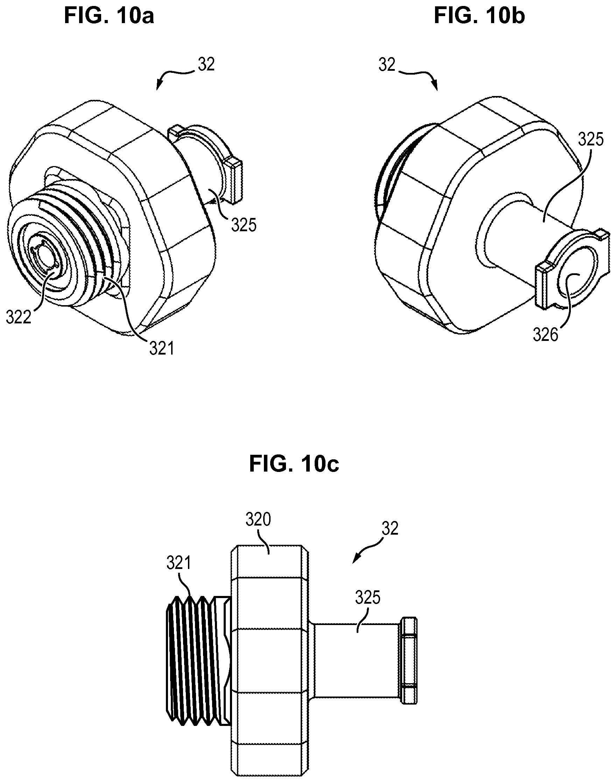

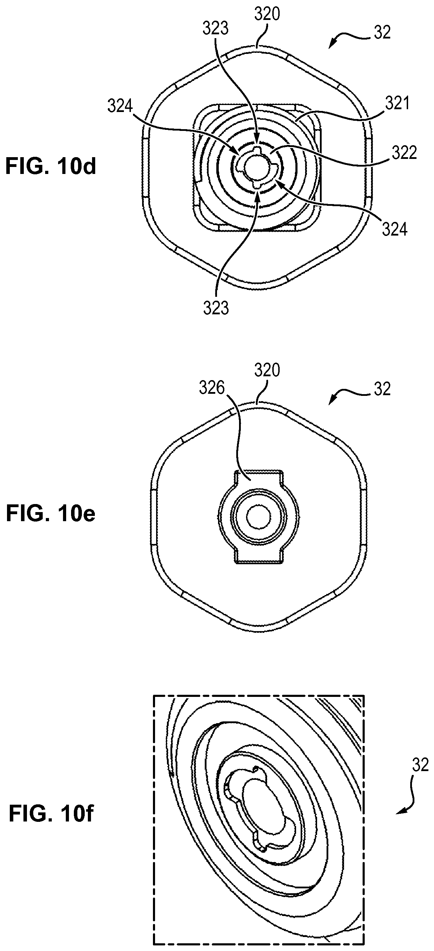

Le module d'entrée 31 est visible sur les **figures 9a, 9b, 9c, 9d et 9e** et module de sortie 32 est visible sur les **figures 10a, 10b, 10c****,** **10d, 10e** et **10f****.**

Le module d'entrée 31 comprend un logement 311 formant la partie femelle 310 pour recevoir la partie mâle 321 du module de sortie 32. Si les modules d'entrée 31 et de sortie 32 sont vissés, alors la partie femelle est taraudée tandis que la partie mâle est filetée.

Pour amener le fluide dans le compartiment, le module d'entrée 31 comprend une tige creuse 312 prolongée par un cône 313. La tige creuse 312 et le cône 313 sont reliés à la partie femelle 310 et permettent d'amener le fluide depuis l'orifice 314 d'entrée du module d'entrée 31.

L'orifice 314 d'entrée présente une forme adaptée pour être relié à un canal permettant d'amener le fluide à analyser. La partie femelle 310 présente la forme d'un cylindre creux connecté au cône 313. La forme conique permet d'avoir au niveau du module d'entrée une grande quantité de flux.

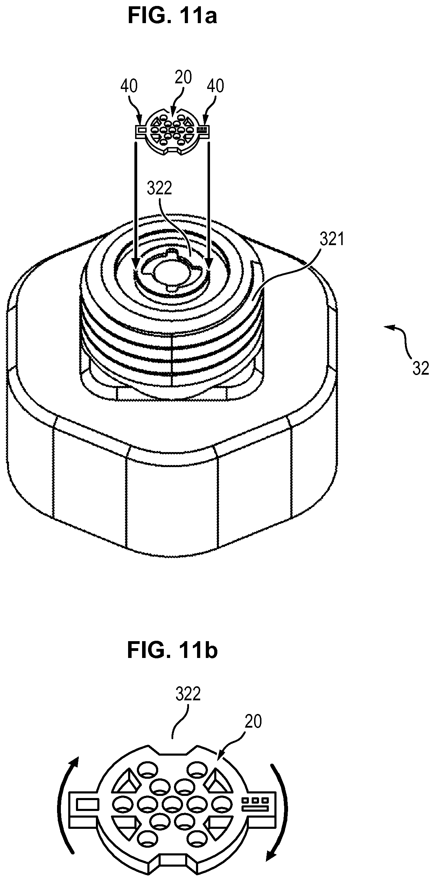

La partie mâle 321 du module de sortie 32 comprend un emplacement 322 configuré pour recevoir les moyens de filtration 20. Cet emplacement 322 présente une forme adaptée à la forme des moyens de filtration 20 qui comprennent à ce titre des pattes 40 (voir la figure 2) permettant leur insertion et leur maintien dans l'emplacement 322. Les moyens de filtration 20 s'insèrent dans l'emplacement 322 en introduisant les pattes dans logements 323 complémentaires prévus au niveau de l'emplacement 322 puis en appliquant une rotation aux moyens des filtration 20 ces derniers sont mis en place pour rester immobile. L'emplacement 322 comprend des gorges 324 permettant l'insertion des pattes pour bloquer les moyens de filtration après rotation. Ces gorges sont usinées de part et d'autre des logements 323 recevant les pattes 40 des moyens de filtration 20. Des contacts électriques peuvent être prévus dans les gorges. Les **figures 11a** et **11b** illustrent les moyens de filtration 20 agencés dans l'emplacement 322.

Pour permettre la sortie du fluide après passage dans les moyens de filtration 20, le module de sortie 32 comprend une tige creuse 325 connectée à un cylindre creux 320. Le cylindre creux 320 est entre la partie male 321 et la tige creuse 320. La tige creuse 325 présente un orifice de sortie 326 adapté pour être relié à un canal permettant d'évacuer le fluide après analyse.

Lorsque les modules d'entrée et de sortie sont assemblés, les moyens de filtration sont à l'intérieur du compartiment et ne sont pas visibles de l'extérieur.

Le compartiment présente l'avantage de pouvoir être facilement démonté pour pouvoir remplacer les moyens de filtration au besoin.

Lorsque les moyens de filtration sont agencés dans le compartiment on obtient un ensemble monobloc.

Le compartiment est en matériau de préférence biocompatible notamment lorsqu'il est destiné à être utilisé *in vivo.*

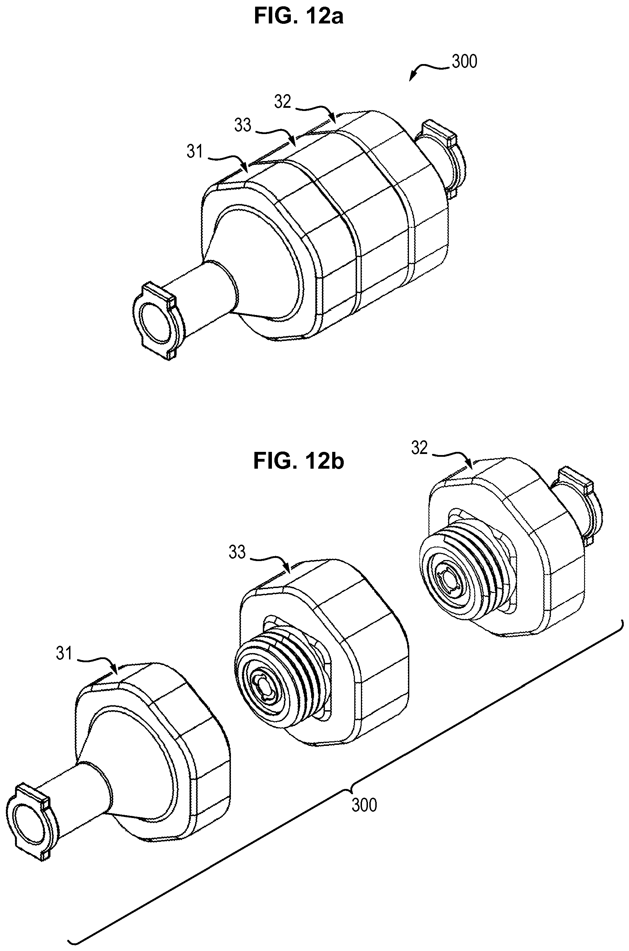

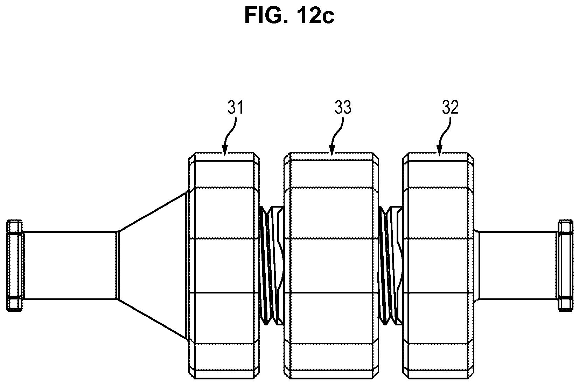

Selon un mode de réalisation de l'invention, comme illustré sur les **figures 12a, 12b** et **12c**, les moyens de filtration peuvent être mis en série. Ainsi, il y a plusieurs membranes filtrantes en série chacune ayant des caractéristiques propres à la capture d'un type de cellule. Alternativement, la membrane peut être définie pour capturer plusieurs types de cellules. Dans ce cas, la membrane comprend des pores de différentes tailles et formes.

La mise en série consiste à assembler plusieurs modules entre eux.

Sur la figure 12a de gauche à droite on a donc un module d'entrée 31 comme décrit ci-avant, un ou plusieurs module(s) intermédiaire(s) 33 et un module de sortie 32 comme décrit également ci-avant.

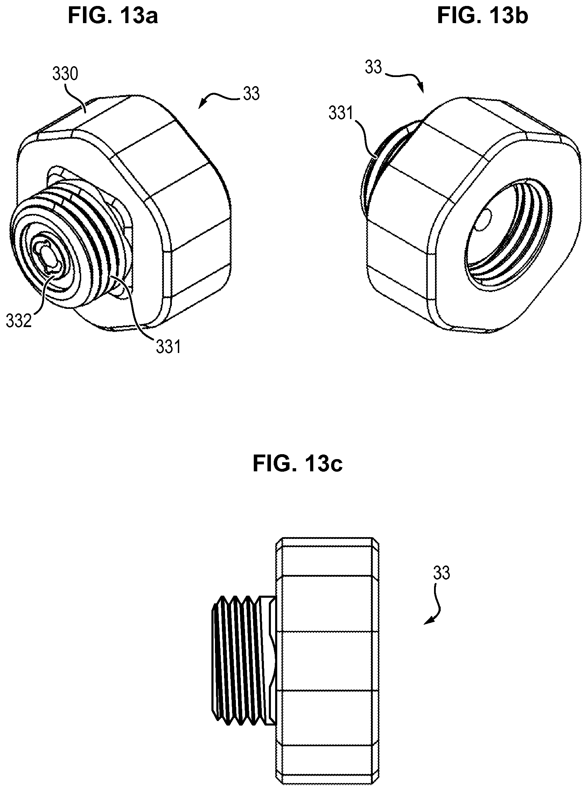

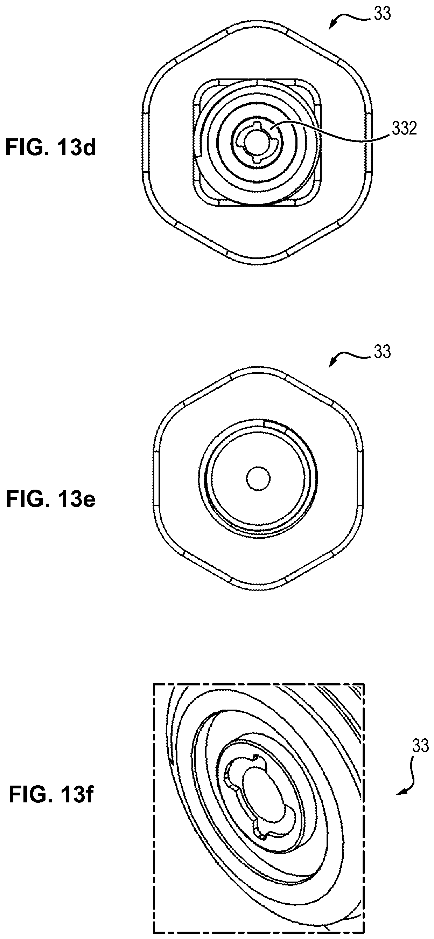

Le module intermédiaire est visible sur les **figures 13a, 13b, 13c****,** **13d, 13e** et **13f****.**

Comme on peut le voir sur ces figures, le module intermédiaire comprend une partie mâle 331 et une partie femelle 330. Le module intermédiaire 33 peut être vissé ou emboité dans/avec respectivement la partie femelle du module d'entrée 31 et la partie mâle du module de sortie 32. Dans le cas où le module intermédiaire 33 est vissé, il comprend une partie mâle filetée et une partie femelle taraudée.

La partie mâle 331 comprend un emplacement 332 pour recevoir des moyens de filtration 20. L'emplacement 332 présente les mêmes formes et caractéristiques que celui du module de sortie 32 précédemment décrit.

En résumé, le module intermédiaire 33 diffère essentiellement du module de sortie en ce qu'il ne comprend pas de tige pour la sortie du fluide.

Grâce à la mise en place d'un ou plusieurs module(s) intermédiaire(s) il est donc possible d'avoir en série plusieurs moyens de filtration pour ainsi capturer et détecter plusieurs types de cellules.

Également, on peut prévoir des modules qui permettent de modifier le fluide en fonction des besoins. Par exemple, un module peut concentrer le fluide avant d'être filtré par les moyens de filtration.

On obtient ainsi un système modulaire pouvant s'adapter à plusieurs types de cellules.

### Compartiment du second type

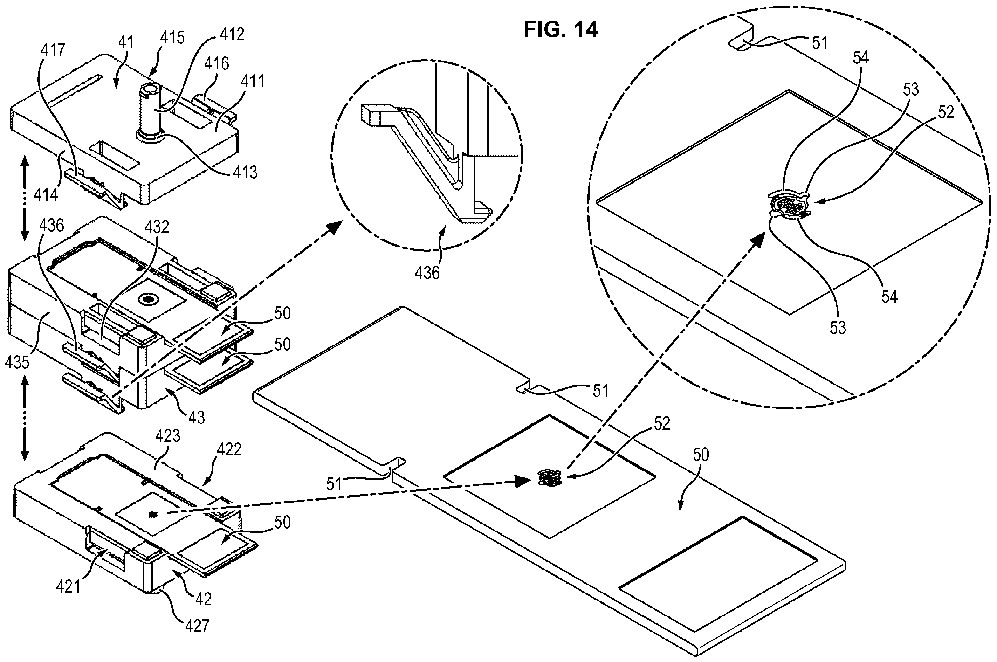

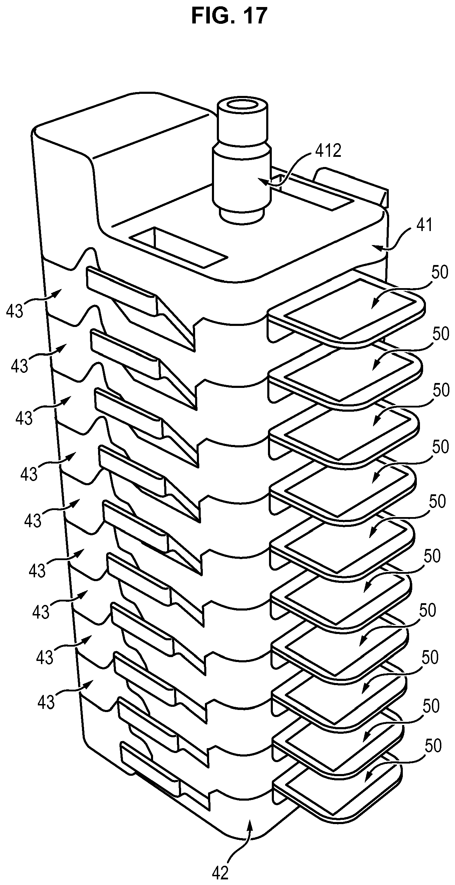

Selon un mode de réalisation illustré sur les **figures 14****,** **15****,** **16** et **17****,** le système de capture et de détection comprend avantageusement un rack d'entrée 41 et un rack de sortie 42 et éventuellement un ou plusieurs rack(s) intermédiaire(s) 43 disposé(s) entre le rack d'entrée 41 et le rack de sortie 42 pour mettre en série plusieurs moyens de filtration 40 comme ci-avant.

Les moyens de filtration 20 sont logés dans le rack de sortie 42 et le cas échéant les racks intermédiaires 43.

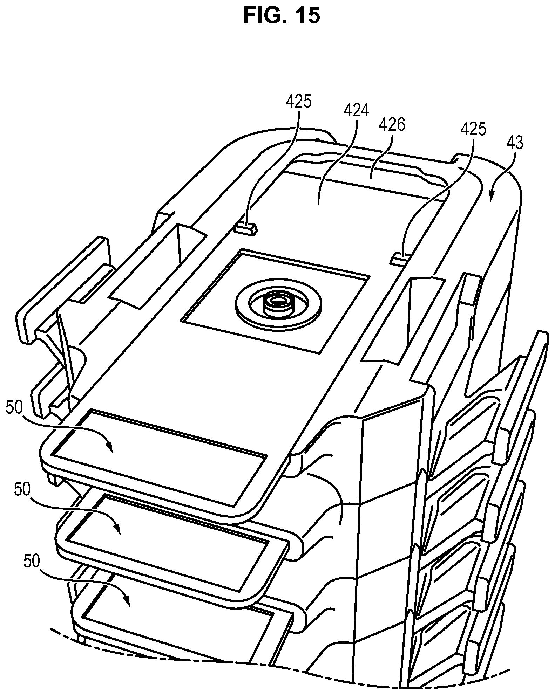

Les racks 41, 42, 43 sont tels qu'ils peuvent s'emboiter entre eux pour former une pile de racks (voir la figure 15).

Le rack d'entrée 41 est parallélépipèdique et comporte une face supérieure 411 comprenant une tige creuse 412 qui s'étend depuis un orifice 413. Ceci permet d'amener le fluide à analyser. Ce rack d'entrée 41 se connecte au rack de sortie 42 ou à un rack intermédiaire 43. Le rack d'entrée 41 comprend sur ses faces latérales 414, 415 des pattes flexibles 416, 417 qui permettent de clipser le rack d'entrée 41 dans des logements complémentaires 421, 422, 431, 432 du rack de sortie 42 ou du rack intermédiaire 43 auquel il est connecté.

Le rack d'entrée 41 se clipse au rack intermédiaire 43 ou au rack de sortie 41 afin que la face inférieure 418 du rack d'entrée repose sur la face supérieure 423, 433 du rack intermédiaire ou de sortie.

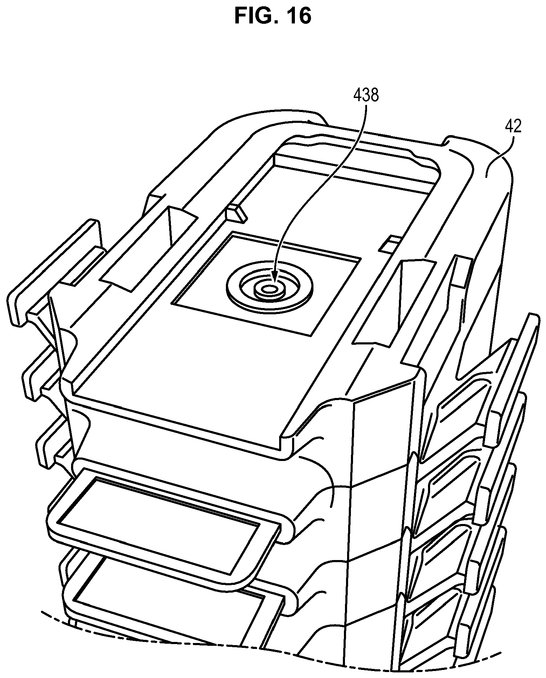

Le rack de sortie 42 est parallélépipèdique et comprend sur sa face supérieure 423 une rainure 424 formant logement pour accueillir une lame 50 de celle du type utilisée pour les microscopes par exemple (voir la figure 16).

Le logement 424 comporte deux ergots 425 situés l'un en face l'autre qui permettent de maintenir en position la lame 50. A ce titre, la lame 50 qui se présente sous la forme d'une plaque comporte des rainures 52 complémentaires aux ergots 425. La lame 50 s'insère dans le logement 424 en prenant appui sur un espace d'accueil 426 formé au fond du logement 424 La lame 50 est alors abaissée pour reposer dans le logement 424. Le rack de sortie 42 comporte un orifice situé sur sa face inférieure 427 situé en son centre par exemple pour laisser passer le fluide à analyser. L'orifice est prolongé par une tige 428 similaire à celle utilisée avec le rack d'entrée 41. Cette tige peut se connecter à un canal qui permet d'évacuer le fluide après passage dans les moyens de filtration 20.

Le rack intermédiaire 43 est parallélépipèdique et comprend sur sa face supérieure 433 une rainure 434 formant logement pour accueillir une lame 50 de celle du type utilisée pour les microscopes par exemple (voir la figure 16). La face supérieure du rack intermédiaire 43 est similaire à celle du rack de sortie 42. Le rack intermédiaire 43 étant destiné à être disposé entre deux racks, la face inférieure du rack intermédiaire comprend sur ses faces latérales 435 des pattes flexibles 436, 437 qui permettent de se clipser dans des logements complémentaires 421, 432 du rack auquel il doit être connecté. Le rack intermédiaire 43 comprend un orifice central 438 pour permettre de laisser passer le fluide.

Lorsque plusieurs racks sont empilés le fluide à analyser circule dans plusieurs racks et donc passe par plusieurs moyens de filtration 20 supportés par la lame 50 propre à chaque rack. La lame 50 comporte un emplacement 52 qui présente une forme adaptée à la forme des moyens de filtration 20 qui comprennent à ce titre des pattes 40 (voir la figure 2) permettant leur insertion et leur maintien dans l'emplacement. Les moyens de filtration 20 s'insèrent dans l'emplacement 52 en introduisant les pattes dans des logements complémentaires 53 prévus au niveau de l'emplacement 52 puis en appliquant une rotation aux moyens des filtration 20 ces derniers sont mis en place pour rester immobile. L'emplacement 52 comprend des gorges 54 permettant l'insertion des pattes pour bloquer les moyens de filtration 20 après rotation. Ces gorges sont usinées de part et d'autre des logements 52 recevant les pattes 40 des moyens de filtration 20. Des contacts électriques peuvent être prévus dans les gorges.

L'intérêt du compartiment du deuxième type est qu'il permet de retirer facilement la lame 50 des racks.

En outre, comme déjà évoqué, l'utilisation d'au moins un rack intermédiaire en plus du rack de sortie permet d'avoir plusieurs membranes filtrantes en série chacune ayant des caractéristiques propres à la capture d'un type de cellule. Alternativement, la membrane peut être définie pour capturer plusieurs types de cellules. Dans ce cas, la membrane comprend des pores de différentes tailles et formes.

Les racks ici décrits sont parallélépipèdiques mais peuvent prendre d'autres formes : cylindriques notamment.

### Compartiment du troisième type

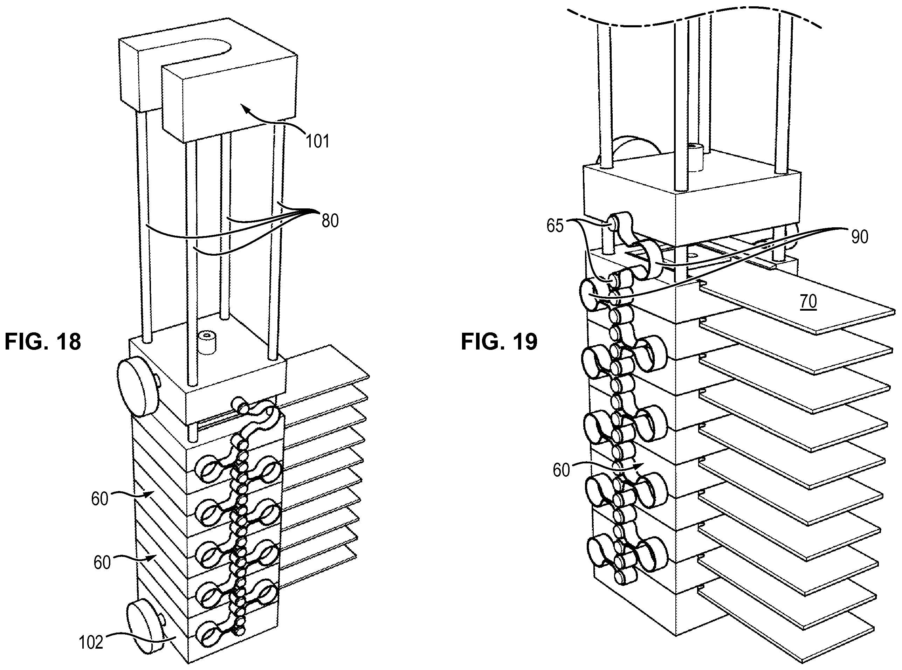

Selon un mode de réalisation illustré sur les **figures 18, 19** **et** **20** on peut prévoir plusieurs racks 60 empilés les uns sur les autres avec une lame 70 similaire à celle décrite ci-dessus. Ces racks 60 prennent la forme de blocs comprenant un emplacement 61 pour insérer la lame 70 sur laquelle on insère des moyens de filtration 20. La lame 70 s'insère dans des rainures 62. Un orifice 63 au centre du bloc permet de laisser s'écouler le fluide comme déjà décrit.

A la différence des racks déjà décrits, au lieu de clipser les racks entre eux et pour améliorer la tenue mécanique de l'ensemble formé par tous les racks, les racks sont maintenus par des tiges 80 formant un bloc de racks. De préférence, quatre tiges 80 sont prévues et traversent les racks 70 qui comprennent à ce titre des orifices 64 dans les coins des racks 70. Pour maintenir les racks 70 des clinquants 90 formés par des lames métalliques maintiennent deux à deux les racks 70. Les clinquants 90 agissent comme des ressorts et permettent de libérer les racks facilement une fois qu'il n'y a plus de contraintes exercées sur eux. Les clinquants sont fixés à deux racks empilés par l'intermédiaire de bras 65 qui dépassent des côtés de chaque rack 70. En effet, pour maintenir ensemble les racks malgré les clinquants, on prévoir des blocs 101, 192 de maintien qui enserrent la colonne de rack, un bloc de maintien supérieur 101 et un bloc de maintien inférieur 102.

Dans le cas des compartiments des deuxième et troisième types, chaque lame peut éventuellement supporter plusieurs moyens de filtration sur un même plan afin d'avoir plusieurs types de cellules capturés par une même lame ou un plus grand nombre de cellules capturées sur la même lame.

### Procédé

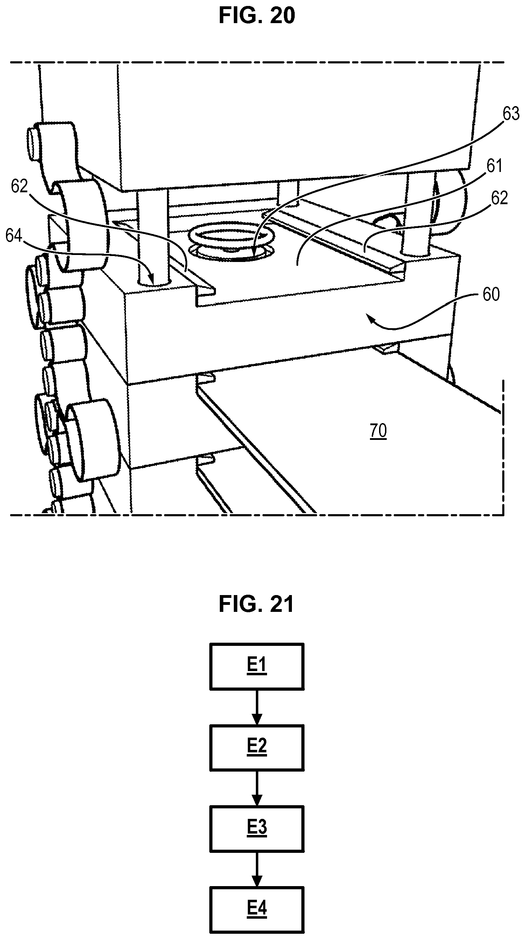

Selon un aspect, l'invention concerne un procédé de capture et de détection de cellules circulantes dans un fluide en relation avec la **figure 21**.

Un procédé de capture peut comprendre une étape d'application d'un signal électrique aux électrodes présentant une fréquence telle qu'un champ électrique crée par les électrodes permet de capturer ou libérer des cellules capturées dans les pores grâce à la force diélectrophorétique générée entre les électrodes.

Pour la capture et la détection, un fluide est mis en circulation dans le système ci-dessus décrit (étape E1).

Un procédé de capture peut comprendre une étape d'application d'un signal électrique aux électrodes présentant une fréquence telle qu'un champ électrique crée par les électrodes permet de capturer des cellules capturées dans les pores grâce à la force diélectrophorétique générée entre les électrodes (étape E2)

De manière avantageuse, la fréquence de polarisation induit une force diélectrophorétique positive pendant la capture de manière à centrer les cellules entre les électrodes et à retenir les cellules dans les pores.

Pour détacher les cellules, un fluide est mis en circulation dans le système ci-dessus décrit dans un sens inverse à celui utilisé pour la capture (étape E3).

Après la capture, il est avantageux de pouvoir détacher des cellules. Ainsi, le procédé comprend une étape d'application d'un signal électrique aux électrodes présentant une fréquence telle qu'un champ électrique crée par les électrodes permet de libérer des cellules capturées dans les pores grâce à la force diélectrophorétique générée entre les électrodes (étape E4).

Pour ce faire la fréquence de polarisation induit une force diélectrophorétique négative permettant de détacher toutes les cellules capturées, la fréquence étant typiquement de 1MHz.

Après la capture et pour détacher uniquement les cellules tumorales, la fréquence induit une force diélectrophorétique permettant de détacher sélectivement un type de cellule, la fréquence étant comprise entre 50 kHz et 150kHz, de préférence 100kHz pour détacher les cellules tumorales.

Également après la capture, la fréquence de polarisation est augmentée par palier entre 10kHz et 200 kHz de manière à détacher des cellules à des instants différente en fonction de leurs propriétés diélectriques.

## Revendications

1. Système de détection d'au moins une espèce présente dans un fluide biologique de préférence d'au moins une cellule circulante ou d'agrégat de cellules présent(e)(s) dans un fluide biologique humain ou animal et notamment de cellules tumorales circulantes (CTC) présentes dans un fluide sanguin, le système de détection comprenant des moyens de filtration (20) dudit fluide, lesdits moyens de filtration (20) comprenant un support plan (24) supportant une membrane filtrante, ladite membrane filtrante comprenant des pores (22) adaptés pour retenir une espèce d'un type donné présente dans le fluide, lesdits moyens de filtration (20) comprenant en outre au moins une ouverture (23) formée dans le support plan (24) en périphérie des pores (22), l'ouverture étant adaptée pour assurer, en fonctionnement au sein du fluide biologique, une continuité de circulation du fluide biologique au travers du système même lorsque au moins un pore (22) est occupé.

2. Système selon la revendication 1, comprenant plusieurs ouvertures en forme de secteur angulaire autour de la membrane filtrante (21).

3. Système selon la revendication 2, comprenant une ouverture centrale formée au centre du support plan (24).

4. Système selon l'une des revendications précédentes, comprenant en outre une pluralité d'électrodes (26) disposées autour dudit au moins un pore (22), lesdites électrodes formant un ou plusieurs circuits électriques polarisés par un signal électrique alternatif permettant la mesure des variations d'impédances complexes entre ces électrodes, dès lors qu'une ou des cellules se logent dans ou à proximité d'un pore (22), la mesure de la variation du module de l'impédance complexe et de sa phase permettant de discriminer le type de cellules.

5. Système selon la revendication précédente, comprenant en outre une inductance (151) connectée aux électrodes de manière à former un circuit résonateur électromagnétique, les électrodes et l'inductance formant un circuit de détection, de préférence interrogeable à distance, de la présence de cellules piégées.

6. Système selon l'une des revendications précédentes, dans lequel les pores (22) de la membrane sont arrangés par groupe de plusieurs pores, chaque groupe présentant un motif, les groupes pouvant être reliés entre eux via une rangée de pores, le motif formé par un groupe présente, de préférence, une forme : hexagonale, circulaire.

7. Système selon l'une des revendications précédentes, comprenant un compartiment (30) dans lequel sont logés les moyens de filtration (20), le compartiment (30) comprenant un module d'entrée (31) et un module de sortie (32) assemblés entre eux pour permettre de faire un circuler un fluide depuis le module d'entrée vers le module de sortie en passant par les moyens de filtration (20).

8. Système selon l'une des revendications 1 à 7, comprenant un rack d'entrée (41) et un rack de sortie (42), une lame (50) supportant les moyens de filtration (20), le rack d'entrée (41) et le rack de sortie (42) étant assemblés entre eux de telle sorte que la lame (50) soit entre le rack d'entrée (41) et le rack de sortie (42) pour permettre de faire un circuler un fluide depuis le rack d'entrée vers le rack de sortie (42) en passant par les moyens de filtration (20).

9. Ensemble de capture comprenant une pluralité de systèmes selon l'une des revendications 7 ou 8 disposés en série, chaque système comprenant des moyens de filtration adaptés pour retenir un type d'espèce.

10. Procédé de capture de cellules circulantes dans un fluide, comprenant une étape de circulation d'un fluide dans un système selon l'une des revendications 4 ou 5, le procédé comprenant une étape d'application d'un signal électrique aux électrodes présentant une fréquence telle qu'un champ électrique crée par les électrodes permet de capturer ou libérer des cellules capturées dans les pores grâce à la force diélectrophorétique générée entre les électrodes.

11. Procédé de capture selon la revendication précédente, dans lequel
- la fréquence de polarisation induit une force diélectrophorétique positive pendant la capture de manière à centrer les cellules entre les électrodes et à retenir les cellules dans les pores.

12. Procédé de capture selon la revendication 10, dans lequel la fréquence de polarisation induit une force diélectrophorétique négative permettant de détacher toutes les cellules capturées, la fréquence étant typiquement de 1MHz.

13. Procédé de capture selon la revendication 10, dans lequel la fréquence induit une force diélectrophorétique permettant de détacher sélectivement un type de cellule, la fréquence étant comprise entre 50 kHz et 150kHz, de préférence 100kHz pour détacher les cellules tumorales.

14. Procédé de capture selon la revendication 10, dans lequel la fréquence de polarisation est augmentée par paliers entre 10kHz et 200 kHz de manière à détacher les cellules capturées à des instants différents en fonction de leurs propriétés diélectriques.

## Patentansprüche

1. System zum Detektieren von mindestens einer Spezies, die in einem biologischen Fluid, vorzugsweise von mindestens einer zirkulierenden Zelle oder einem Zellaggregat, die/das in einem menschlichen oder tierischen biologische Fluid vorhanden ist/sind, und insbesondere von zirkulierenden Tumorzellen (CTC), die in einem Blutfluid vorhanden sind, wobei das Detektionssystem Filtermittel (20) für das Fluid umfasst, wobei die Filtermittel (20) einen ebenen Träger (24) umfassen, der eine Filtermembran trägt, wobei die Filtermembran Poren (22) umfasst, die geeignet sind, eine in dem Fluid vorhandene Spezies eines bestimmten Typs zurückzuhalten, wobei die Filtermittel (20) ferner mindestens eine Öffnung (23) umfassen, die in dem ebenen Träger (24) an der Peripherie der Poren (22) ausgebildet ist, wobei die Öffnung geeignet ist, im Betrieb innerhalb des biologischen Fluids eine kontinuierliche Zirkulation des biologischen Fluids durch das System auch dann zu gewährleisten, wenn mindestens eine Pore (22) besetzt ist.

2. System nach Anspruch 1, das mehrere winkelsektorförmige Öffnungen um die Filtermembran (21) herum umfasst.

3. System nach Anspruch 2, das eine zentrale Öffnung umfasst, die in der Mitte des ebenen Trägers (24) ausgebildet ist.

4. System nach einem der vorstehenden Ansprüche, das ferner eine Vielzahl von Elektroden (26) umfasst, die um die mindestens eine Pore (22) herum angeordnet sind, wobei die Elektroden einen oder mehrere elektrische Stromkreise bilden, die durch ein elektrisches Wechselsignal polarisiert sind, das die Messung der Änderungen komplexer Impedanzen zwischen diesen Elektroden ermöglicht, sobald sich eine oder mehrere Zellen in oder in der Nähe einer Pore (22) ansiedeln, wobei die Messung der Änderung des Moduls der komplexen Impedanz und ihrer Phase die Unterscheidung des Zelltyps ermöglicht.

5. System nach vorstehendem Anspruch, das ferner eine Induktanz (151) umfasst, die mit den Elektroden derart verbunden ist, dass ein elektromagnetischer Resonanzkreis gebildet wird, wobei die Elektroden und die Induktanz einen vorzugsweise aus der Ferne abfragbaren Detektionskreis bezüglich des Vorhandenseins eingefangener Zellen bilden.

6. System nach einem der vorstehenden Ansprüche, wobei die Poren (22) der Membran in Gruppen aus mehreren Poren angeordnet sind, wobei jede Gruppe ein Muster aufweist, wobei die Gruppen miteinander über eine Porenreihe verbunden sein können, wobei das von einer Gruppe gebildete Muster vorzugsweise eine sechseckige, kreisförmige Form aufweist.

7. System nach einem der vorstehenden Ansprüche, das ein Abteil (30) aufweist, in dem die Filtermittel (20) untergebracht sind, wobei das Abteil (30) ein Eingangsmodul (31) und ein Ausgangsmodul (32) umfasst, die miteinander verbunden sind, um ein Fluid vom Eingangsmodul durch die Filtermittel (20) zum Ausgangsmodul zirkulieren zu lassen.

8. System nach einem der vorstehenden Ansprüche, umfassend ein Eingangsgestell (41) und ein Ausgangsgestell (42), ein Plättchen (50), das die Filtermittel (20) trägt, wobei das Eingangsgestell (41) und das Ausgangsgestell (42) derart miteinander verbunden sind, dass sich das Plättchen (50) zwischen dem Eingangsgestell (41) und dem Ausgangsgestell (42) befindet, um ein Fluid vom Eingangsgestell zum Ausgangsgestell (42) durch die Filtermittel (20) zirkulieren zu lassen.

9. Einfanganordnung, umfassend eine Vielzahl von seriell angeordneten Systemen nach einem der Ansprüche 7 oder 8, wobei jedes System Filtermittel umfasst, die zum Zurückhalten eines Speziestyps geeignet sind.

10. Verfahren zum Einfangen von zirkulierenden Zellen in einem Fluid, das einen Schritt des Zirkulierens eines Fluids in einem System nach einem der Ansprüche 4 oder 5 umfasst, wobei das Verfahren einen Schritt des Anlegens eines elektrischen Signals an die Elektroden mit einer Frequenz umfasst, die derart ist, dass ein von den Elektroden erzeugtes elektrisches Feld es ermöglicht, in den Poren eingefangene Zellen dank der zwischen den Elektroden erzeugten dielektrophoretischen Kraft einzufangen oder freizugeben.

11. Einfangverfahren nach vorstehendem Anspruch, wobei
- die Polarisationsfrequenz während des Einfangens eine positive dielektrophoretische Kraft induziert, um die Zellen zwischen den Elektroden zu zentrieren und die Zellen in den Poren zurückzuhalten.

12. Einfangverfahren nach Anspruch 10, wobei die Polarisationsfrequenz eine negative dielektrophoretische Kraft induziert, die es ermöglicht, alle eingefangenen Zellen zu lösen, wobei die Frequenz typischerweise 1 MHz beträgt.

13. Einfangverfahren nach Anspruch 10, wobei die Frequenz eine dielektrophoretische Kraft induziert, die es ermöglicht, einen Zelltyp selektiv zu lösen, wobei die Frequenz zwischen 50 kHz und 150 kHz, vorzugsweise 100 kHz, liegt, um Tumorzellen zu lösen.

14. Einfangverfahren nach Anspruch 10, wobei die Polarisationsfrequenz stufenweise zwischen 10 kHz und 200 kHz derart erhöht wird, dass die eingefangenen Zellen entsprechend ihren dielektrischen Eigenschaften zu unterschiedlichen Zeitpunkten gelöst werden.

## Claims

1. System for detecting at least one species present in a biological fluid, preferably at least one circulating cell or cell aggregate present in a human or animal biological fluid, and in particular circulating tumor cells (CTC) present in a blood fluid, the detection system comprising filtration means (20) for said fluid, said filtration means (20) comprising a planar support (24) supporting a filter membrane, said filter membrane comprising pores (22) adapted to retain a species of a given type present in the fluid, said filtration means (20) further comprising at least one aperture (23) formed in the planar support (24) at the periphery of the pores (22), the aperture being adapted to ensure, in operation within the biological fluid, continuity of flow of the biological fluid through the system even when at least one pore (22) is occupied.

2. System according to claim 1, comprising a plurality of angular sector-shaped openings around the filter membrane (21).

3. System according to claim 2, comprising a central aperture formed in the center of the planar support (24).

4. System according to one of the preceding claims, further comprising a plurality of electrodes (26) arranged around said at least one pore (22), said electrodes forming one or more electrical circuits polarized by an alternating electrical signal allowing the measurement of variations in complex impedances between these electrodes, whenever one or more cells are housed in or near a pore (22), the measurement of the variation in the modulus of the complex impedance and its phase making it possible to discriminate the type of cells.

5. System according to the preceding claim, further comprising an inductor (151) connected to the electrodes so as to form an electromagnetic resonator circuit, the electrodes and inductor forming a circuit for detecting, preferably remotely interrogating, the presence of trapped cells.

6. System according to one of the preceding claims, in which the pores (22) of the membrane are arranged in groups of several pores, each group presenting a pattern, the groups being interconnectable via a row of pores, the pattern formed by a group preferably having a shape: hexagonal, circular.

7. System according to one of the preceding claims, comprising a compartment (30) in which the filtration means (20) are housed, the compartment (30) comprising an inlet module (31) and an outlet module (32) interconnected to enable fluid to be circulated from the inlet module to the outlet module via the filtration means (20).

8. System according to one of claims 1 to 7, comprising an inlet rack (41) and an outlet rack (42), a blade (50) supporting the filtration means (20), the inlet rack (41) and the outlet rack (42) being assembled together so that the blade (50) is between the inlet rack (41) and the outlet rack (42) to enable a fluid to flow from the inlet rack to the outlet rack (42) via the filtration means (20).

9. Capture assembly comprising a plurality of systems according to one of claims 7 or 8 arranged in series, each system comprising filtration means adapted to retain one type of species.

10. Method of capturing circulating cells in a fluid, comprising a step of circulating a fluid in a system according to one of claims 4 or 5, the method comprising a step of applying an electrical signal to electrodes having a frequency such that an electric field created by the electrodes enables cells captured in the pores to be captured or released by virtue of the dielectrophoretic force generated between the electrodes.

11. Method of capturing according to the preceding claim, in which
- the polarization frequency induces a positive dielectrophoretic force during capture so as to center the cells between the electrodes and retain the cells in the pores.

12. Method of capturing according to claim 10, in which the polarization frequency induces a negative dielectrophoretic force enabling all captured cells to be detached, the frequency typically being 1MHz.

13. Method of capturing according to claim 10, in which the frequency induces a dielectrophoretic force for selectively detaching one cell type, the frequency being between 50kHz and 150kHz, preferably 100kHz for detaching tumor cells.

14. Method of capturing according to claim 10, in which the polarization frequency is increased in steps between 10kHz and 200kHz so as to detach the captured cells at different times depending on their dielectric properties.
